# EUROPEAN PATENT SPECIFICATION

(11) **EP 2 214 659 B1**
(45) Date of publication and mention of the grant of the patent: **31.08.2011**
(21) Application number: 08856548.6
(22) Date of filing: 06.11.2008
(51) Int. Cl.: A61K 31/192, A61K 31/341, A61K 31/36, A61P 37/06, A61P 37/02, A61P 1/06, A61P 19/02, A61P 17/06, A61P 7/06, A61P 35/00

(54) **USE OF 4-OXOBUTANOIC ACID DERIVATIVES IN THE TREATMENT OF PATHOLOGIES ASSOCIATED WITH IMMUNOLOGICAL DISORDERS**
VERWENDUNG VON 4-OXOBUTANSÄURE-DERIVATEN BEI DER BEHANDLUNG VON MIT IMMUNOLOGISCHEN LEIDEN VERBUNDENEN ERKRANKUNGEN
UTILISATION DE DÉRIVÉS DE L'ACIDE 4-OXOBUTANOÏQUE DANS LE TRAITEMENT DE PATHOLOGIES ASSOCIÉES À DES TROUBLES IMMUNOLOGIQUES

(30) Priority: 03.12.2007 EP 07291449
(43) Date of publication of application: 11.08.2010
(73) Proprietor: Merck Patent GmbH, 64293 Darmstadt (DE)
(72) Inventor: AUTIER, Valérie, F-91190 Gif sur Yvette (FR); FABREGUE, Roland, F-01700 Neyron (FR); MIOSSEC, Patrick, F-75013 Paris (FR)
(86) International application number: PCT/EP2008/009384
(87) International publication number: WO 2009/071161

(56) References cited:
- EP-A- 1 369 114
- WO-A-00/06560
- WO-A-97/17317
- WO-A-99/61413
- WO-A-03/047561
- WO-A-2004/060368
- WO-A-2005/035497
- WO-A-2007/081878
- US-A- 5 886 022
- US-A1- 2004 236 147
- PANAJOTOVA V ET AL: "Pharmacological profile of the novel potent antirheumatic 4-(2',4'-difluorobiphenyl-4-yl)-2-methyl-4 -oxobutanoic acid" ARZNEIMITTEL FORSCHUNG. DRUG RESEARCH, ECV EDITIO CANTOR VERLAG, AULENDORF, DE, vol. 47, no. 5, 1 May 1997 (1997-05-01), pages 648-652, XP008101583 ISSN: 0004-4172
- NICOLAUS B J R: "Symbiotic Approach to Drug Design" DECISION MAKING IN DRUG RESEARCH, XX, XX, 1 January 1983 (1983-01-01), pages 173-186, XP002197412

## Description

The present invention relates to the use of a 4-oxobutanoic acid derivative according to claim 1 for the preparation of a pharmaceutical composition for the treatment or prevention of pathologies associated with immunological disorders according to claim 1

Autoimmunity is a natural phenomenon which corresponds to a tolerance of the immune system. The breaking of tolerance mechanisms leads to the destructive action of the immune system on the natural constituents of the body, following the proliferation of self-attacking B or T lymphocytes of strong affinity, leading to a wide variety of pathologies collectively known as autoimmune diseases. Autoimmune diseases are distinguished as specific for organs, such as type 1 diabetes, autoimmune thyroiditis, autoimmune hepatopathies, myasthenia, autoimmune bullous diseases, vitiligo, autoimmune uveitis, autoimmune retinitis and autoimmune cytopaenia, or systemic or non-organ-specific autoimmune diseases, such as systemic lupus erythematosus, Gougerot-Sjögren syndrome, rheumatoid arthritis, scleroderma, polymyositis and dermato-polymyositis, mixed connective tissue disease, isolated angeitis and atrophic polychondritis. Some cancers also originate from a disorder of immunological type (Muller et al., Current Cancer Drug Targets (2007), 7(1), 31-40; Baniyash, Michal Seminars in Cancer Biology (2006), 16(1), 80-88). These diseases tend to appear within the same family, indicating that autoimmune diseases are dependent, beside environmental factors, on immunogenetic factors.

The treatment of autoimmune diseases often involves a strategy of non-specific immunosuppression usually combining corticotherapy and antimitotics. These treatments are not without side effects, which restrict their application to "severe" autoimmune diseases. These therapeutic strategies are aimed at acting selectively on overactivated lymphocytes or on self-reacting clones.

During cell differentiation and clone expansion induced by the antigen, the T cells secrete various lymphokines, especially interleukin 2 (IL2), which are necessary to the amplification of the immune response and the proliferation of the effector cells. Immunosuppressive treatments inhibit this reaction and each agent uses a prevailing action on one of the steps of the immune response.

Various classes of agents are currently used clinically:
- analogues of purine (Azathioprine (Imurel) and mercaptopurine) and pyrimidine bases
- cyclosporin (Sandimmun, neoral)
- tacrolimus and rapamycin
- folic acid analogues (methotrexate)
- glucocorticoids
- monoclonal antibodies

The therapeutic arsenal currently used by clinicians is not devoid of problems for patients, stemming from the nature of the side effects observed, such as nephrotoxicity (renal failure) and neurotoxicity (tremors, headaches, anxiety, insomnia) for tacrolimus, and hepatotoxicity and hyperurecaemia for cyclosporin.

The establishment of new immunosuppression therapies is justified in all pathologies involving inappropriate activation of the immune system, such as the autoimmune diseases (type 1 diabetes, rheumatoid arthritis, lupus erythematosus, haemolytic anaemia, Crohn's disease, psoriasis, some rheumatic complaints) and in transplantations in order to maintain the survival of the transpplant in transplantees and/or to prevent the transplant-versus-host reaction.

Tryptophan is an essential amino acid that the body obtains through ingested proteins. Tryptophan derived from food first reaches the liver via the hepato-portal system, where some of it is used for protein synthesis. The unused part is distributed in the other organs via the blood stream or metabolised in the kynurenine pathway, mainly in the liver (review in Moffett and Namboodiri, 2003). Tryptophan is also the only source of amino acids for several very important molecules, such as serotonin and melatonin. Moreover, if the supply of niacin from food is inadequate to provide for the synthesis of nicotinamide adenine dinucleotide (NAD), the metabolism of tryptophan becomes an alternative source of this essential cofactor. It has recently been demonstrated that tryptophan metabolism is closely involved in inflammatory reactions and immunological disorders linked to many diseases. More particularly, the role of tryptophan metabolites originating from the kynurenine pathway has been described. The main enzymes and substrates for tryptophan metabolism in the kynurenine pathway have been described. The liver is the only organ known to possess all of the enzymes enabling metabolism in the various segments of this pathway.

More recently, it has been demonstrated that high concentrations of tryptophan could lead to an accumulation of leukocytes, reflecting its likely implication in the immune system (Gross et al., 1999). Gamma-interferon is a proinflammatory cytokine released by activated T cells or other leukocytes, leading to the production of free radicals by macrophages and neutrophils (Tennenberg et al., 1993). During an immune response, gamma-interferon induces the catabolism of tryptophan in a strong and sustained manner (Grant et al., 2000). Among the key metabolites originating from this catabolism are kynurenine, 3-hydroxy-anthranilate and quinolinate, whose role as immunomodulators has been described (Moffett et al. 1993, 1994)

The use of tryptophan metabolites, such as kynurenine, for the treatment of any disease which requires immunosuppressive treatment is described in EP 1 369 114 A.

4-Oxobutanoic acid derivatives have already been described in patent application WO 98/07 681 as antidiabetic agents and more particularly for the treatment of non-insulin-dependent diabetes. They have also been described as inhibitors of a tryptophan metabolism enzyme, kynurenine 3-hydrolase (WO 2004/060 368 and WO 2004/060 369).

This chemical family of the general formula (I) is also known for the preparation of a medicament for the treatment of inflammation (WO 2003/047 561).

The present patent application relates to the use of a compound chosen from
- (-) 2-benzyl-4-(4-fluorophenyl)-4-oxobutanoic acid
- (+) 2-benzyl-4-(4-fluorophenyl)-4-oxobutanoic acid,
- solvates and salts of these acids
for the preparation of a medicament for the treatment of a pathology associated with immunological disorders, selected from Crohn's disease, psoriasis, lupus erythematosus, haemolytic anaemia.

The present invention also encompasses the tautomeric forms of the compounds, the enantiomers, diastereoisomers and epimers of these compounds, and also their solvates.

Examples of salts of the compounds include pharmacologically acceptable salts, such as the sodium salts, potassium salts, magnesium salts, calcium salts, amine salts and other salts of the same type (aluminium, iron, bismuth).

The compounds were subjected to biological tests for demonstrating their immunosuppressive activity.

The compounds of the invention can be formulated together with any appropriate excipient, in any form suitable for enteral (more particularly oral) or parenteral administration, for example in the form of tablets, gel capsules, powders, sugar-coated tablets, or drinkable or injectable solutions. These suitable forms and excipients are as defined in patent application WO 98/7681 filed by the applicants.

The compounds can be administered to adults in daily doses between about 0.001 mg and 400 mg orally or between 0.001 and 100 mg parenterally.

### - Experimental part:

The immunosuppressive activity was evaluated by studying the humoral immunity in rats through the quantification of direct haemolytic plaques ("plaque-forming cells" - PFC) after immunisation with sheep erythrocytes.

This technique consists in stimulating the immune system with an antigenic agent, the sheep erythrocytes, and in evaluating the effect of a substance on the immune response. This immune response is evaluated by measuring the proportion of splenocytes that produce anti-sheep erythrocyte antibodies in the presence of a complement (Jerne N.K., 1974).

The effect of the compounds, in suspension in methylcellulose hydrogel (Sigma ref: M0512), on the immune system was evaluated in male Wistar rats after oral administration for 5 and 7 days (5 days before sensitisation and 2 days after). The administration volume was 10 ml/kg.

10 rats were used in each group, each group corresponding to a dose of a compound, plus a control group (blank) and a positive control group (reference substance). The reference substance used was cyclophosphamide (monohydrate) from Sigma (ref: C0768) at a dose of 20 mg/kg administrated subcutaneously at a rate of 1 ml/kg, only administrated on the fifth day. During the four previous days, the animals were treated with the compound vehicle (methylcellulose).

On the fifth day, one hour after treatment, the animals were sensitised with sheep erythrocytes administrated intravenously (caudal vein), at a rate of 0.5 ml per animal. The sheep erythrocytes were suspended at a concentration of 2x10⁹ cells/ml in a sodium chloride solution (isotonic).

On the ninth day, i.e. 4 days after sensitisation, the spleen was removed after euthanasia by exsanguination (section of the abdominal aorta) of the animals, which were previously anaesthetised with pentobarbital (Sanofi, 60 mg/kg).

Half of the spleen was broken up (ground), giving, after preparation, a suspension of splenocytes in PBS buffer (Dulbecco Gibco Ref: 3018199) containing 10⁷ cells per millilitre.

The haemolytic plaque (PFC) reaction was induced by addition of 50 microlitres of the splenocyte suspension obtained for each animal in 200 microlitres of a suspension of PBS comprising complement and sheep erythrocytes.

The mixture, placed between the slide (holder) and cover slip creating an evaluation chamber (40 µl) delimited by paraffin, was incubated for one hour at 37°C.

At the end of the incubation, the slides were immediately placed flat at 4°C. The evaluation was performed as soon as possible. The number of haemolytic plaques (PFC) was determined by a standardised method of slide evaluation using an optical microscope. A haemolytic plaque (PFC) is a plaque where only one trapped splenocyte can be observed. The number of haemolytic plaques (PFC) is calculated for 10⁶ spleen cells.

The experimental results with molecule A are presented in Table I. Molecule A: (-) 4-(4-Fluorophenyl)-4-oxo-2-phenylmethylbutanoic acid

**Table I**

| 1st experiment ^{a} | % inhibition |
|---|---|
| Cyclophosphamide | |
| (20 mg/Kg) | 85 ** |
| A (30 mg/Kg) | 3 |
| A 100 mg/Kg) | 19 ** |
| A (300 mg/Kg) | 29 ** |
| 2^{nd} experiment^{b} | % inhibition |
| Cyclophosphamide | |
| (20 mg/Kg) | 54 ** |
| A (0.01 mg/Kg) | 0 |
| A (0.1 mg/Kg) | 43 * |
| A (1 mg/Kg) | 42 ** |

| | |
|---|---|
| P<0.05; *P<0.01 ^{a} treatment for 5 days before sensitisation ^{b} treatment for 7 days (5 days before sensitisation and 2 days after) | |

Under these experimental conditions using the haemolytic plaque (PFC - plaque forming cells) method, Example A induces a decrease in the humoral immune response, which is a statistically significant decrease in the number of haemolytic plaques (PFC) at and above 0.1 mg/kg. These results also show that compound A has a stronger effect after the sensitisation phase.

## Claims

1. Use of a compound chosen from
- (-) 2-benzyl-4-(4-fluorophenyl)-4-oxobutanoic acid
- (+) 2-benzyl-4-(4-fluorophenyl)-4-oxobutanoic acid,
- solvates and salts of these acids
for the preparation of a medicament for the treatment of a pathology associated with immunological disorders, selected from Crohn's disease, psoriasis, lupus erythematosus, haemolytic anaemia.

## Patentansprüche

1. Verwendung einer Verbindung, die aus
- (-)-2-Benzyl-4-(4-fluorphenyl)-4-oxobutansäure,
- (-)-2-Benzyl-4-(4-fluorphenyl)-4-oxobutansäure,
- Solvaten und Salzen dieser Säuren
ausgewählt ist, zur Herstellung eines Arzneimittels für die Behandlung einer Pathologie, die in Zusammenhang mit immunologischen Erkrankungen steht, die aus Morbus Crohn, Psoriasis, Lupus erythematodes, hämolytischer Anämie ausgewählt sind.

## Revendications

1. Utilisation d'un composé choisi parmi
- l'acide (-)-2-benzyl-4-(4-fluorophényl)-4-oxobutanoïque,
- l'acide (+)-2-benzyl-4-(4-fluorophényl)-4-oxobutanoïque,
- les solvats et les sels de ces acides,
pour la préparation d'un médicament destiné au traitement d'une pathologie associée à des troubles immunologiques, choisis parmi la maladie de Crohn, le psoriasis, le lupus érythémateux et l'anémie hémolytique.
